# EUROPEAN PATENT APPLICATION

(11) **EP 1 911 433 A1**
(43) Date of publication of application: **16.04.2008**
(21) Application number: 06021539.9
(22) Date of filing: 13.10.2006
(51) Int. Cl.: A61K 6/00, A61K 6/087

(54) **Dental obturator point**

(71) Applicant: Dentsply DeTrey GmbH, 78467 Konstanz (DE)
(72) Inventor: Klee, Joachim E., 78315 Radolfzell (DE)
(74) Representative: Hartz, Nikolai

(57) **Abstract**

Dental obturator point having an obturation body of a material comprising
(a) a polymer blend comprising
(a1) 5 to 95 wt% based on the total amount of the polymer blend of a thermoplastic epoxide amine addition polymer, and
(a2) 95 to 5 wt% based on the total blend of a high performance polymer; and
(b) an particulate-filler;

whereby the obturation body has a radio opacity of at least 3 mm/mm Al.

## Description

### Field of the invention

The present invention relates to a dental obturator point having an obturation body with high strength, flexibility, radio opacity and compatibility with a root canal sealant. The present invention further related to a method for producing a dental obturator point, a polymer blend and the use of the blend for the preparation of an obturator point.

### Background of the Invention

Dental obturator points are used by the dental practitioner to fill a prepared root canal. Given the small dimensions of the root canal and the fact that root canals are frequently not straight, but shaped with turns and screw-like portions, any obturator point used for dental purposes must provide a combination of high strength and flexibility. Moreover, given that radio opacity of the dental obturator point is essential in subsequent diagnostic methods, obturator points are required to contain significant amounts of radio opaque material such as specific inorganic fillers.

US 5,118,297 discloses a dental obturator point having an obturation body of a plastic material such as UDEL polysulfone MG-11 (AMOCO Performance Products, Inc) or Vectra VC-3 (Hoechst Celanese Corp.) optionally containing powdered tungsten as an inorganic particulate filler.

The dental obturator point of US 5,118,297 is problematic in that the material suggested for the obturation body is excessively stiff. Accordingly, the obturator point may only be used for filling a root canal after a softening step has been performed wherein the material is subjected to heating. Moreover, the material is not compatible with a root canal sealant whereby an adhesive bond between the sealant and the obturation body cannot be formed. Finally, the properties of the material suggested by US 5,118,297 render any mass production of the dental obturator point costly and difficult. In particular, the melting point of the material is typically in a range of at least 250°C whereby molding techniques are difficult to employ for the preparation of dental obturator points.

EP-A 1 545 571 discloses a dental root canal filling material which may be used for the preparation of dental root canal filling cones having high flexibility. The material disclosed by EP-A 1 545 571 has a low Tg and a decomposition temperature which is typically below 300 °C. Accordingly, the material is not suitable for the preparation of dental obturator points having a combination of high stiffness and low brittleness.

It is therefore an object of the present invention to provide a dental obturator point having an obturation body with high strength, excellent flexibility, high radio opacity and compatibility with a root canal sealant, whereby the dental obturator point may be easily produced. Moreover, the dental obturator points should preferably fulfill the requirements of ISO 6877, especially with regard to the brittleness test and the required radio-opacity of at least 6 compared to 1 mm aluminium.

### Summary of the Invention

The present invention provides dental obturator point having an obturation body of a material comprising
(a) a polymer blend comprising
   (a1) 5 to 95 wt% based on the total amount of the polymer blend of a thermoplastic epoxide amine addition polymer, and
   (a2) 95 to 5 wt% based on the total blend of a high performance polymer; and
(b) an particulate filler;
whereby the obturation body has a radio opacity of at least 3 mm/mm Al.

The present invention provides also a method for producing a dental obturator point, which comprises the following steps:
(i) providing a composition comprising
   (a) a polymer blend comprising
      (a1) 5 to 95 wt% based on the total amount of the polymer blend of a thermoplastic epoxide amine addition polymer, and
      (a2) 95 to 5 wt% based on the total blend of a high performance polymer; and
   (b) an particulate filler; and
(ii) molding the composition to provide a dental obturator point having a radio opacity of at least 3 mm/mm Al.

Furthermore, the present invention provides a polymer blend comprising
(a1) 5 to 95 wt% based on the total amount of the polymer blend of a thermoplastic epoxide amine addition polymer, and
(a2) 95 to 5 wt% based on the total blend of a high performance polymer.

Finally, the present invention provides the use of a composition comprising
(a) a polymer blend comprising
   (a1) 5 to 95 wt% based on the total amount of the polymer blend of a thermoplastic epoxide amine addition polymer, and
   (a2) 95 to 5 wt% based on the total blend of a high performance polymer; and
(b) an particulate filler,
for preparing a dental obturating point having a radio opacity of at least 3 mm/mm Al.

### Detailed Description of the Invention

The dental obturator point according to the invention has an obturation body. The size and shape of the obturation body is adapted to the typical sizes and shapes of a root canal. In view of the variation of the shapes and sizes of root canals, dental obturator points may be combined in a kit of parts wherein one or more dental obturator points with an obturation body of a specific size and shape are combined with one or more obturator points with an obturation body of a different specific size and shape. The dental obturator point according to the invention may comprise further portions such as handle portions or neck portions integrally attached to the obturation body. The material of any optional further portions may be the same or different as the material of the obturation body. The obturation body has a radio opacity of at least 3 mm/mm Al. Preferably, the radio opacity is at least 5 mm/mmAl, more preferably at least 6 mm/mmAl.

The material of the obturation body comprises a polymer blend and a particulate filler.

The polymer blend comprises 5 to 95 wt% based on the total amount of the polymer blend of a thermoplastic epoxide amine addition polymer, and 95 to 5 wt% based on the total blend of a high performance polymer. Preferably, the polymer blend comprises 10 to 45 wt% based on the total amount of the polymer blend of a thermoplastic epoxide amine addition polymer, and 90 to 65 wt% based on the total blend of a high performance polymer. The thermoplastic epoxide amine addition polymer and the high performance polymer may be a single type of polymer or a blend of different types of polymers, respectively.

The thermoplastic epoxide amine addition polymer preferably has a T_{g} of at most 30 °C. More preferably, the thermoplastic epoxide amine addition polymer has a T_{g} of at most 10°C.

In a preferred embodiment, the thermoplastic epoxide amine polymer is obtainable by reacting
(a) one mole of a compound of the following formula (I) 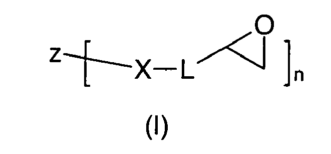 wherein
   Z represents
      an divalent C₂₋₄₂ hydrocarbon group, which groups may contain 1 to 6 oxygen atoms, and which may be substituted by 1 to 6 C₁₋₄ alkyl groups;
   X independently represents
      a single bond or
      an oxygen atom or a nitrogen atom substituted by a C₁₋₆ alkyl group;
   L independently represents
      a single bond or
      an optionally substituted C₁₋₁₆ alkylene group,
      an optionally substituted C₆₋₁₄ arylene group,
      an optionally substituted C₇₋₁₆ alkylenearylene group,
      an optionally substituted C₇₋₁₆ arylenealkylene group,
      which groups may be substituted by 1 to 6 C₁₋₄ alkyl groups; and
      n represents 2; and
(b) at least n moles of one or more compounds
   (b1) of the following formula (II) 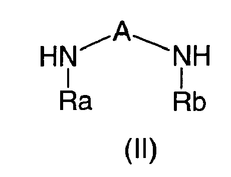 wherein
      A represents a divalent saturated aliphatic C₂₋₁₆ hydrocarbon group or a divalent saturated cycloaliphatic C₃₋₆ hydrocarbon group, which groups may contain 1 to 6 oxygen atoms, and which may be substituted by 1 to 6 C₁₋₄ alkyl groups;
      Rₐ and R_{b} are the same or different and represent a hydrogen atom, a C₁₋₆ alkyl or a C₃₋₁₄ cycloalkyl group, which may be substituted by one or more members of the group selected from a C₁₋₄ alkyl group, C₁₋₄ alkoxy group, a benzyl group, and a hydroxy group; or
   (b2) of the following formula (III)

      R'NH₂ (III)
wherein R' represents
a substituted or unsubstituted C₁ to C₁₈ alkyl group,
a substituted or unsubstituted C₃ to C₁₈ cycloalkyl group,
a substituted or unsubstituted C₇ to C₃₀ aralkyl group,
which groups may be substituted by one or more members of the group selected from a C₁₋₄ alkyl group, C₁₋₄ alkoxy group, and a hydroxy group,
optionally in combination with up to 2 weight% based on the total weights of the reactants of a further di- or polyamine compound.

In the formula (I), Z is an n-valent C₂₋₄₂ hydrocarbon group optionally containing 1 to 6 oxygen atoms, and which may be substituted by 1 to 6 C₁₋₄ alkyl groups. Preferably, Z is an n-valent C₂₋₂₂ hydrocarbon group. Z is divalent (n=2) so that thermoplastic polymers are obtained. The hydrocarbon group may be substituted by 1 to 6 C₁₋₄ alkyl groups. Specific examples of the alkyl groups are methyl, ethyl, n-propyl, i-propyl, n-butyl, i-butyl or tert.-butyl. The hydrocarbon group may contain 1 to 6 oxygen atoms in the hydrocarbon group in the form of aliphatic or aromatic ether bonds, keto groups, carboxylic acid groups, hydroxyl groups, or ester groups. Specifically, Z may be a divalent substituted or unsubstituted C₁ to C₁₈ alkylene group, substituted or unsubstituted C₆₋₁₄ arylene group, substituted or unsubstituted C₃ to C₁₈ cycloalkylene group, substituted or unsubstituted C₇ to C₃₀ arylenealkylenearylene group. Preferably, Z represents a saturated aliphatic C₂₋₁₈ hydrocarbon chain which may contain 2 to 4 oxygen atoms, and which may be substituted by 1 to 6 C₁₋₄ alkyl groups, or Z may be a substituted or unsubstituted C₇ to C₃₀ arylenealkylenearylene group which may be substituted by 1 to 6 C₁₋₄ alkyl groups.

In formula (I), X represents a single bond or an oxygen atom or a nitrogen atom substituted by a C₁₋₆ alkyl group. Preferably, X is an oxygen atom.

In formula (I), L may be a single bond or an optionally substituted C₁₋₁₆ alkylene group, an optionally substituted C₆₋₁₄ arylene group, an optionally substituted C₇₋₁₆ alkylenearylene group, an optionally substituted C₇₋₁₆ arylenealkylene group, which groups may be substituted by 1 to 6 C₁₋₄ alkyl groups. Examples for a C₁₋₁₆ alkylene group are methylen, ethylene, propylene or butylene. Examples for a C₆₋₁₄ arylene group are p-phenylene or m-phenylene. Examples for a C₇₋₁₆ alkylenearylene group are -(CH₂)ₓC₆H₅-, wherein x is an integer of from 1 to 6. Examples for a C₇₋₁₆ arylenealkylene group are -C₆H₅(CH₂)ₓ-, wherein x is an integer of from 1 to 6. Preferably, L is an optionally substituted C₁₋₁₆ alkylene group, in particular a methylene goup.

In a preferred embodiment, X is an oxygen atom and/or L is an alkylene group, preferably a methylene group, and/or X-L is -OCH₂-.

In formula (II), A is a divalent saturated aliphatic C₂₋₁₆ hydrocarbon group or a divalent saturated cycloaliphatic C₃₋₆ hydrocarbon group, which groups may be based on linear or branched alkylene groups having 2 to 16 carbon atoms, preferably 4 to 10 carbon atoms, or cycloalkylene groups having 3 to 6 carbon atoms, preferably 4 to 6 carbon atoms. The hydrocarbon group may be substituted by one or more C₁₋₄ alkyl groups. Specific examples of the alkyl groups are methyl, ethyl, n-propyl, i-propyl, n-butyl, i-butyl or tert.-butyl. The hydrocarbon group may contain 1 to 6 oxygen atoms in the carbon chain connecting the amino groups or in a side chain. Preferably the divalent saturated aliphatic C₂₋₁₆ hydrocarbon group or the divalent saturated cycloaliphatic C₃₋₆ hydrocarbon group is highly flexible due to the presence of ether bonds and the absence of bulky groups. In a preferred embodiment, A is a divalent group based on a straight chain alkylene group which may contain ether bonds. In a preferred embodiment, A may be -(CH₂)₂O(CH₂)₂O(CH₂)₂-.

R^{a} and R^{b} may the same or different and represent a hydrogen atom, a C₁₋₆ alkyl or a C₃₋₁₄ cycloalkyl group. Examples for a C₁₋₆ alkyl group can include linear or branched alkyl groups having 1 to 6 carbon atoms, preferably 1 to 4 carbon atoms, for example, methyl, ethyl, n-propyl, isopropyl, n-butyl, isobutyl, sec-butyl, tert-butyl, n-pentyl, isopentyl and n-hexyl. Examples of the C₃₋₁₄ cycloalkyl group can include those having 3 to 14 carbon atoms, for example, cyclopropyl, cyclobutyl, cyclopentyl and cyclohexyl. The C₁₋₆ alkyl group and the C₃₋₁₄ cycloalkyl group may optionally be substituted by one or more members of the group selected from a C₁₋₄ alkyl group, C₁₋₄ alkoxy group, a phenyl group, and a hydroxy group. Examples for a C₁₋₄ alkyl group can include linear or branched alkyl groups having 1 to 4 carbon atoms, for example, methyl, ethyl, n-propyl, isopropyl, n-butyl, isobutyl, sec-butyl, tert-butyl. Examples for an C₁₋₄ alkoxy group can include linear or branched alkoxy groups having 1 to 4 carbon atoms, for example, methoxy, ethoxy, n-propoxy, isopropoxy, n-butoxy, isobutoxy, sec-butoxy, and tert-butoxy. Preferably, R^{a} and R^{b} are hydrogen.

In the preparation of the prepolymer, the compound of formula (II) may be used in combination with an amine compound of the formula RNH₂, wherein R represents C₁₋₆ alkyl or a C₃₋₁₄ cycloalkyl group, which may be substituted by one or more members of the group selected from a C₁₋₄ alkyl group, C₁₋₄ alkoxy group, a phenyl group, and a hydroxy group, or a further di- or polyamine compound. The amine of the formula R^{a}NH₂ and/or the further di- or polyamine compound may be used to replace up to n/1.5 moles, preferably n/20 to n/2 moles of the compound of formula (II) used in the reaction for preparing the prepolymer, wherein n is as defined above.

The preparation of suitable thermoplastic epoxide amine polymers is disclosed in EP-A 1 547 571. The synthesis of secondary amine terminated epoxide-amine prepolymers was described by Klee (Acta Polym. 37(1986) 272; Angew. Makromol. Chem. 147 (1987) 71; Acta Polym. 45 (1994) 73).

Particularly preferred thermoplastic epoxide amine polymer may be represented by one of the following general formulas: 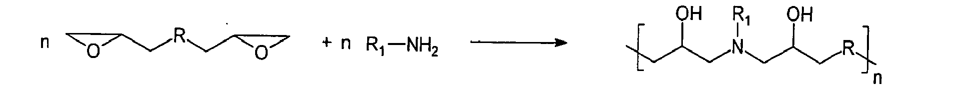 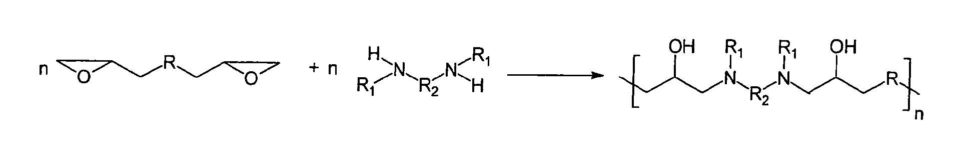 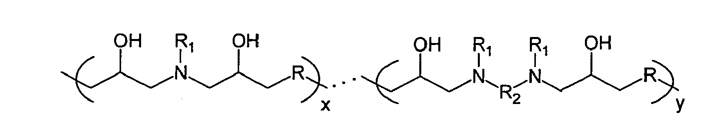 wherein R is a moiety formed from a diepoxide, such as 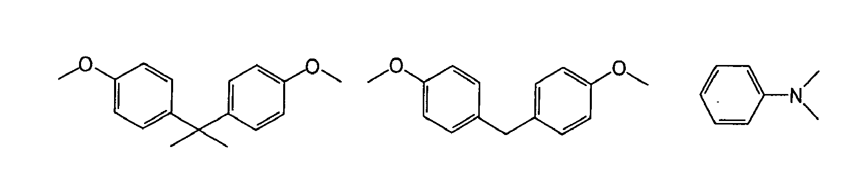 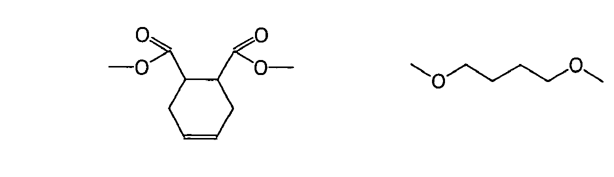
R¹ denotes a monofunctional substituted C₁ to C₁₈ alkylene, a substituted or unsubstituted C₅ to C₁₈ cycloalkylene, a substituted or unsubstituted C₅ to C₁₈ arylene or heteroarylene, such as 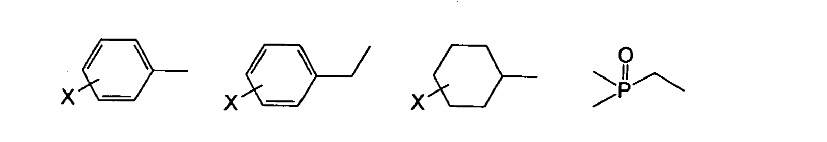 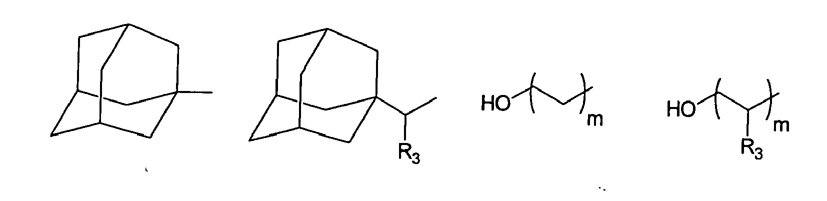
R² denotes a difunctional substituted or unsubstituted C₁ to C₁₈ alkylene, a substituted or unsubstituted C₅ to C₁₈ cycloalkylene, a substituted or unsubstituted C₅ to C₁₈ arylene or heteroarylene, such as 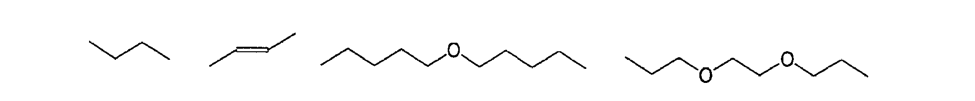 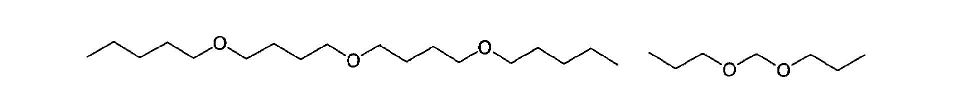 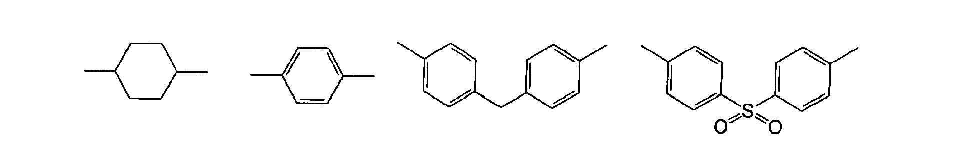
R³ denotes hydrogen or C₁ to C₁₈ alkylene, such as H, CH₃, C₂H₅, C₃H₇ and X is hydrogen or a substituent selected from the group of OCH3, F, Cl, Br, J, CH₃, COCH₃, NO₂, COOC₂H₅.

In a preferred embodiment the epoxide monomer used for the preparation of a thermoplastic epoxide amine polymer is a diepoxide selected from the group of diglycidylethers such as diglycidyl ether of bisphenol-A, diglycidyl ether of bis-phenol-F, butandiol diglycidyl ether, N,N-diglycidylaniline or delta3-tetrahydrophthalic acid diglycidyl ester.

In a preferred embodiment the disecondary diamine monomer used for the preparation of a thermoplastic epoxide amine polymeris selected from the group of N,N'-dibenzyl ethylene diamine, N,N'-dibenzyl-3,6-dioxa-octandiamine-1,8, N,N'-dibenzyl-5-oxanonane diamine-1,9, N,N'-dibenzyl-(2,2,4)/(2,4,4)-trimethylhexamethylene diamine, N,N'-dicyclohexyl ethylene diamine, N,N'-dimethyl-p-xylylene diamine.

In a preferred embodiment the primary monoamine used for the preparation of a thermoplastic epoxide amine polymer is selected from benzylamine, 1-aminoadamantan, a-phenethylamine and ethanol amine.

The polymer blend comprises a high performance polymer. A high performance polymer according to the present invention may have a supermolecular structure characterized by mesophases of the liquid crystal type. In particular the high performance polymer may be a liquid crystalline polymer such as lyotropic liquid crystalline or thermotropic liquid crystal polymers. Lyotropic liquid crystalline polymers are preferred. Specific examples of high performance polymers of the liquid crystalline type are wholly aromatic polyesters and wholly aromatic polyamides. Alternatively, the high performance polymer may be a polymer having a rigid molecular chain such as aromatic polymers selected from polyethers, polyetherketones, polyertherketone ketones.

The high performance polymers used in the present invention are characterized by high mechanical and or thermal properties. Typically, a high performance polymer has a melting point of at least 200° C.

The high performance polymer may be a wholly aromatic polymer selected from polyesters, polyketones, polysulfones, polyetheresters, polyphenylsulfones, polyethersulfones, polyimides and polyamine imides. More specifically, the high-performance polymer may be selected from the group of aromatic polyesters, aromatic polyamides, aromatic polyurethanes, aromatic polyureas, poly(p-phenylene benzthiazol), aromatic polysulfides, aromatic polyether, polyether ketones, polyether ether ketones, polyether ketone ketones or the like.

The high performance polymer may be a polymer according to one of the following formulas

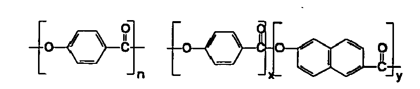

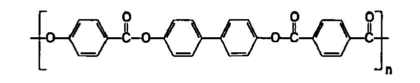

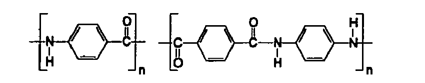

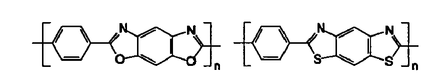

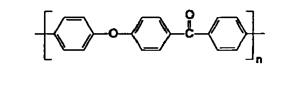

The material of the obturation body of the dental obturator point according to the invention may comprise at least 40 wt.% based on the entire composition of a particulate filler. Preferably, the material comprises at least 50 wt% based on the entire composition of the particulate filler. The filler is preferably a radio-opaque filler selected from an inorganic compound such as La₂O₃, ZrO₂, BiPO₄, CaWO₄, BaWO₄, SrF₂, Bi₂O₃ a metal like W or Bi or organic fillers, such as polymer granulate, splinter polymers or a combination of organic and/or inorganic fillers. The filler is incorporated so as to provide a radio-opacity of at least 3 mm/mm Al, preferably at least 5 to 7 mm/mm Al, most preferably at least 7 mm/mm Al.

The dental obturator point according to the invention may contain additives such as a stabilizer or a plasticizer.

The dental obturator point according to the invention is obtainable by a molding method. A preferred molding technique is injection molding.

The invention will now be further illustrated based on the following Examples and Comparative Examples.

### Example 1

72.183 g (0.212 mol) N,N'-Dibenzyl-5-oxanonandiamin-1,9, 32.274 g (0.212 mol) 1-amino-adamantane and 145.544 g (0.424 mol) Bis-2,2-[4-(2,3-epoxypropoxy)-phenyl]-propane were polymerized for 120 hours at 50 °C. The resulting epoxide-amine addition polymer is soluble in organic solvents like THF, CHCl₃ and CHCl₃/CH₃OH.

250 g of the epoxide-amine addition polymer, 1050 g VECTRA^{™} A-950 and 1400 g tungsten powder were blended with an extruder in the temperature range of 280 - 310 °C.

The obtained thermoplastic composite material was used for injection molding to form root canal cones and cylindric rods for measurement of mechanical properties which are summarized in Table 1.

### Comparative Example 1

250.00 g (0.734 mol) Bis-2,2-[4-(2,3-epoxypropoxy)-phenyl]-propane (Mn 346 g/mol), 33.54 g (0.220 mol) 1-amino-adamantane, 175.05 g (0.51 mol) N,N'-dibenzyl-5-oxanonanediamine-1.9 and 1093.03 g CaWO₄ and 9.85 g Aerosil 200 were mixed homogeneously and polymerized 120 hours at 50 °C. The composition is characterized by following values: radio-opacity RO = 10.1 mm/mm Al and a glass transition temperature of T_{g} = 48 °C.

The obtained thermoplastic composite material was used for injection molding to form root canal cones and cylindric rods for measurement of mechanical properties which are summarized in Table 1.

### Mechanical properties of composite materials

| | | Example 1 | Comparative Example 1 |
|---|---|---|---|
| Flexural strength (FS) | MPa | 150.3 ± 7.3 | 92.1 ± 2.0 |
| Break of rods during measurement of FS | - | 0 of 6 | 0 of 6 |
| E-modulus | MPa | 3860 ± 180 | 4807 ± 707 |
| Compressive strength | MPa | 64.9 ± 6.4 | 63.0 ± 1.4 |
| Yield | MPa | 61.2 ± 7.3 | 58.2 ± 2.5 |
| Bending of ISO 20 sized points after loading of | g | > 134 | > 54 |

## Claims

1. Dental obturator point having an obturation body of a material comprising
(a) a polymer blend comprising
(a1) 5 to 95 wt% based on the total amount of the polymer blend of a thermoplastic epoxide amine addition polymer, and
(a2) 95 to 5 wt% based on the total blend of a high performance polymer; and
(b) an particulate filler;
whereby the obturation body has a radio opacity of at least 3 mm/mm Al.

2. The dental obturator point according to claim 1, wherein the polymer blend comprises
(a1) 10 to 45 wt% based on the total amount of the polymer blend of the thermoplastic epoxide amine addition polymer, and
(a2) 90 to 65 wt% based on the total blend of the high performance polymer.

3. The dental obturator point according to claim 1 or 2, which is obtainable by molding the material.

4. The dental obturator point according to any one of the preceding claims wherein the material of the material of the obturation body comprises at least 40 wt.% of the filler.

5. The dental obturator point according to any one of the preceding claims wherein the thermoplastic epoxide amine addition polymer has a T_{g} of at most 30° C

6. The dental obturator point according to any one of the preceding claims wherein the high performance polymer has a melting point of at least 200° C.

7. The dental obturator point according to any one of the preceding claims wherein the high performance polymer is a wholly aromatic polymer selected from polyesters, polyketones, polysulfones, polyetheresters, polyphenylsulfones, polyethersulfones, polyimides and polyaminimides.

8. The dental obturator point according to any one of the preceding claims wherein the epoxide amine polymer is is obtainable by reacting
(a) one mole of a compound of the following formula (I) 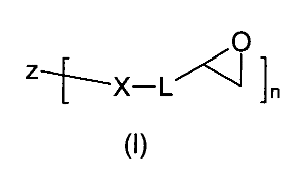 wherein
Z represents
an divalent C₂₋₄₂ hydrocarbon group, which groups may contain 1 to 6 oxygen atoms, and which may be substituted by 1 to 6 C₁₋₄ alkyl groups;
X independently represents
a single bond or
an oxygen atom or a nitrogen atom substituted by a C₁₋₆ alkyl group;
L independently represents
a single bond or
an optionally substituted C₁₋₁₆ alkylene group,
an optionally substituted C₆₋₁₄ arylene group,
an optionally substituted C₇₋₁₆ alkylenearylene group,
an optionally substituted C₇₋₁₆ arylenealkylene group,
which groups may be substituted by 1 to 6 C₁₋₄ alkyl groups; and
n represents 2; and
(b) at least n moles of one or more compounds
(b1) of the following formula (II) 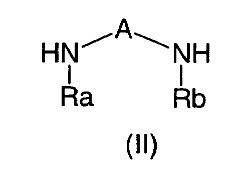 wherein
A represents a divalent saturated aliphatic C₂₋₁₆ hydrocarbon group or a divalent saturated cycloaliphatic C₃₋₆ hydrocarbon group, which groups may contain 1 to 6 oxygen atoms, and which may be substituted by 1 to 6 C₁₋₄ alkyl groups;
Rₐ and R_{b} are the same or different and represent a hydrogen atom, a C₁₋₆ alkyl or a C₃₋₁₄ cycloalkyl group, which may be substituted by one or more members of the group selected from a C₁₋₄ alkyl group, C₁₋₄ alkoxy group, a benzyl group, and a hydroxy group; or
(b2) of the following formula (III)
R'NH₂ (III)
wherein R' represents
a substituted or unsubstituted C₁ to C₁₈ alkyl group,
a substituted or unsubstituted C₃ to C₁₈ cycloalkyl group,
a substituted or unsubstituted C₇ to C₃₀ aralkyl group, which groups may be substituted by one or more members of the group selected from a C₁₋₄ alkyl group, C₁₋₄ alkoxy group, and a hydroxy group,
optionally in combination with up to 2 weight% based on the total weights of the reactants of a further di- or polyamine compound.

9. The dental obturator point according to claim 8, wherein said amine monomer and said epoxide monomer are polymerized to form a polymer within the scope of at least one of the general formulas: 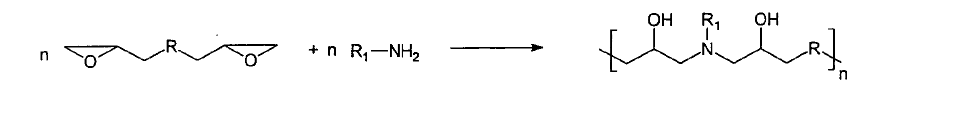 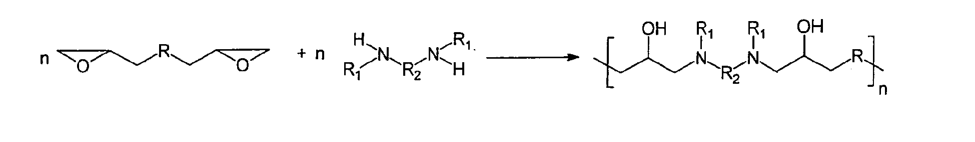 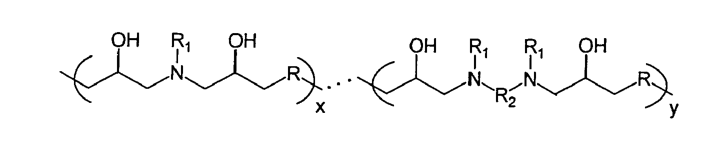 wherein R is a moiety formed from a diepoxide, such as 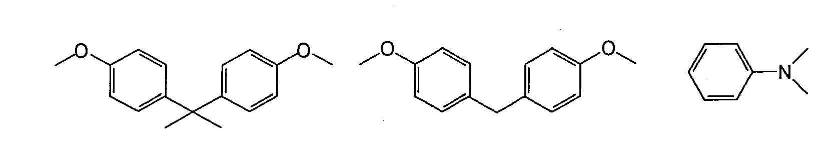 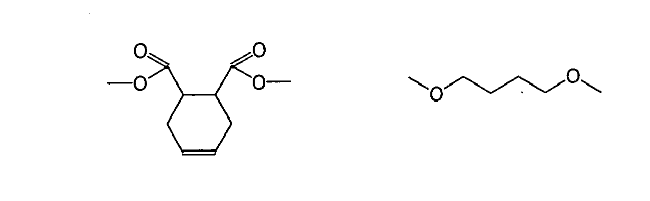
R¹ denotes a monofunctional substituted C₁ to C₁₈ alkylene, a substituted or unsubstituted C₅ to C₁₈ cycloalkylene, a substituted or unsubstituted C₅ to C₁₈ arylene or heteroarylene, such as 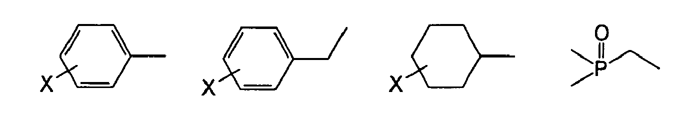 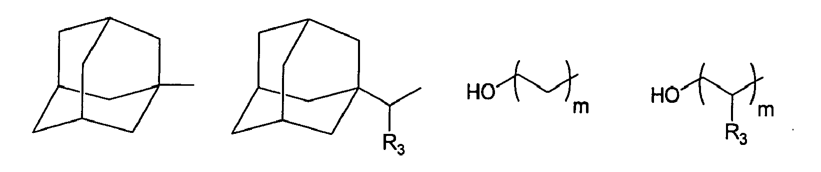
R² denotes a difunctional substituted or unsubstituted C₁ to C₁₈ alkylene, a substituted or unsubstituted C₅ to C₁₈ cycloalkylene, a substituted or unsubstituted C₅ to C₁₈ arylene or heteroarylene, such as 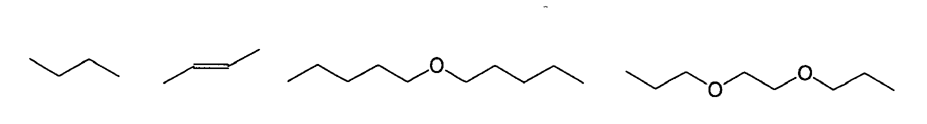 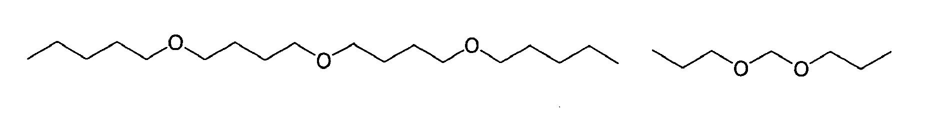 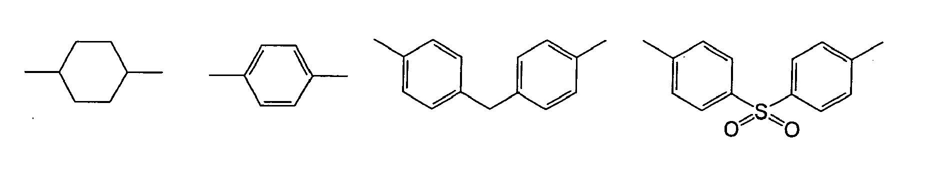
R³ denotes hydrogen or C₁ to C₁₈ alkylene, such as H, CH₃, C₂H₅, C₃H₇ and X is hydrogen or a substituent selected from the group of OCH3, F, Cl, Br, J, CH₃, COCH₃, NO₂, COOC₂H₅.

10. The dental obturator point according to claim 8, wherein said epoxide monomer is a diepoxide selected from the group of diglycidylethers such as diglycidyl ether of bisphenol-A, diglycidyl ether of bis-phenol-F, butandiol diglycidyl ether, N,N-diglycidylaniline or delta3-tetrahydrophthalic acid diglycidyl ester.

11. The dental obturator point according to claim 8, wherein said primary monoamine preferably is benzylamine, 1-aminoadamantan, a-phenethylamine and ethanol amine.

12. The dental obturator point according to claim 8, wherein said disecondary diamine preferably is N,N'-dibenzyl ethylene diamine, N,N'-dibenzyl-3,6-dioxa-octandiamine-1,8, N,N'-dibenzyl-5-oxanonane diamine-1,9, N,N'-dibenzyl-(2,2,4)/(2,4,4)-trimethylhexamethylene diamine, N,N'-dicyclohexyl ethylene diamine, N,N'-dimethyl-p-xylylene diamine.

13. The dental obturator point according to any one of the preceding claims, whereby the high-performance polymer is selected from the group of aromatic polyesters, aromatic polyamides, aromatic polyurethanes aromatic polyureas, poly(p-phenylene benzthiazol), aromatic polysulfides, aromatic polyether, polyether ketones, polyether ether ketones, polyether ketone ketones or similar.

14. The dental obturator point according to any one of the preceding claims, whereby the high-performance polymer is selected from the group of the following polymers:

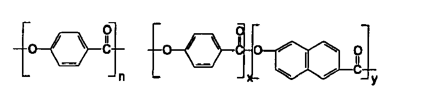

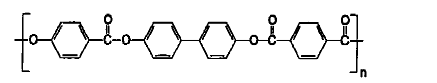

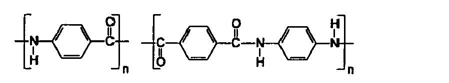

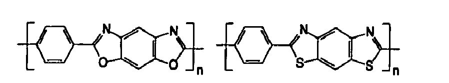

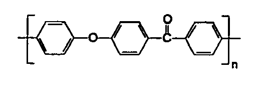

15. The dental obturator point according to any one of the preceding claims, whereby the radio-opaque filler is an inorganic compound such as La₂O₃, ZrO₂, BiPO₄, CaWO₄, BaWO₄, SrF₂, Bi₂O₃ a metal like W or Bi or organic fillers, such as polymer granulate, splinter polymers or a combination of organic and/or inorganic fillers.

16. The dental obturator point according to any one of the preceding claims, containing fillers which provide a radio-opacity of at least 3 mm/mm Al, preferably at least 5 to 7 mm/mm Al, most preferably at least 7 mm/mm Al.

17. The dental obturator point according to any one of the preceding claims, wherein said points contain additives such as stabilizer and plasticizer.

18. A method for producing a dental obturator point, which comprises the following steps:
(i) providing a composition comprising
(a) a polymer blend comprising
(a1) 5 to 95 wt% based on the total amount of the polymer blend of a thermoplastic epoxide amine addition polymer, and
(a2) 95 to 5 wt% based on the total blend of a high performance polymer; and
(b) an particulate filler; and
(ii) molding the composition to provide a dental obturator point having a radio opacity of at least 3 mm/mm Al.

19. Polymer blend comprising
(a1) 5 to 95 wt% based on the total amount of the polymer blend of a thermoplastic epoxide amine addition polymer, and
(a2) 95 to 5 wt% based on the total blend of a high performance polymer.

20. Use of a composition comprising
(a) a polymer blend comprising
(a1) 5 to 95 wt% based on the total amount of the polymer blend of a thermoplastic epoxide amine addition polymer, and
(a2) 95 to 5 wt% based on the total blend of a high performance polymer; and
(b) an particulate filler;
for preparing a dental obturation point having a radio opacity of at least 3 mm/mm Al.
